# EUROPÄISCHE PATENTSCHRIFT

(11) **EP 3 215 599 B1**
(45) Veröffentlichungstag und Bekanntmachung des Hinweises auf die Patenterteilung: **23.06.2021**
(21) Anmeldenummer: 15800732.8
(22) Anmeldetag: 05.11.2015
(51) Int. Cl.: C12M 1/107, B01F 7/00, B01F 3/04, C12M 3/00, C12M 1/06

(54) **RÜHREINRICHTUNG FÜR EINEN FERMENTER EINER BIOGASANLAGE**
STIRRER UNIT FOR A FERMENTER IN A BIOGAS PLANT
DISPOSITIF D'AGITATION POUR UN FERMENTEUR D'UNE INSTALLATION DE BIOGAZ

(30) Priorität: 07.11.2014 DE 102014116242
(43) Veröffentlichungstag der Anmeldung: 13.09.2017
(73) Patentinhaber: UTS Biogastechnik GmbH, 85399 Hallbergmoos (DE)
(72) Erfinder: CZWALUK, Andreas, 49377 Vechta (DE)
(74) Vertreter: BSB Patentanwälte Schütte & Engelen Partnerschaft mbB
(86) Internationale Anmeldenummer: PCT/EP2015/075828
(87) Internationale Veröffentlichungsnummer: WO 2016/071454

(56) Entgegenhaltungen:
- EP-A1- 2 270 128
- EP-A2- 1 895 641
- WO-A1-2007/118788
- WO-A1-2014/020544
- DE-A1- 1 557 076
- DE-A1- 3 818 727
- DE-A1- 3 902 326
- DE-A1- 4 321 722
- DE-A1- 10 303 097
- DE-A1- 19 721 528
- DE-A1-102006 053 339
- DE-A1-102008 010 841
- DE-A1-102009 029 274
- DE-A1-102012 021 206
- DE-B1- 2 823 238
- DE-U1- 29 822 149
- DE-U1-202005 005 165
- DE-U1-202006 010 384
- DE-U1-202009 006 223
- US-A1- 2011 177 558

## Beschreibung

Die vorliegende Erfindung betrifft eine Rühreinrichtung, welche insbesondere, aber nicht nur, für einen Fermenter einer Biogasanlage vorgesehen ist. Eine solche Rühreinrichtung umfasst ein Gehäuse und eine Antriebseinrichtung zum drehbaren Antreiben wenigstens eines Rührflügels der Rühreinrichtung. Die Antriebseinrichtung weist eine Antriebswelle und einen Antriebsmotor auf.

Die DE 15 57 076 A1 offenbart einen Rühreinsatz für einen Rührkessel mit einem Rührwerk mit einer Welle, an deren Enden zwei gegensinnig arbeitenden Rührelemente angebracht sind, um axiale Kräfte weitgehend aufzuheben. Die zu rührende Flüssigkeit wird auch zur Kühlung des Motors und zur Schmierung der Lager eingesetzt.

Biogasanlagen umfassen regelmäßig einen Fermenter oder auch mehrere Fermenter, in die ein Substrat eingebracht wird, um Biogas zu erzeugen. Dabei ist eine Umrührung des in dem Fermenterinnenraum vorhandenen Substrates erforderlich, um günstige Bedingungen für den Betrieb des Fermenters zu erhalten und zu gewährleisten.

Zum Umrühren werden Rührgeräte bzw. Rühreinrichtungen eingesetzt, die regelmäßig einen oder mehrere Rührflügel aufweisen, um das Substrat entsprechend zu durchmischen. Im Stand der Technik sind Rührgeräte bekannt geworden, die hydraulisch angetrieben werden. Derartige Rührgeräte haben den Vorteil, dass innerhalb des Fermenters das Rührgerät für den Betrieb keine Elektrizität benötigt. Das ist vorteilhaft, da sich in dem Fermenterinnenraum gegebenenfalls auch explosionsfähiges Gas bzw. ein explosionsfähiges Gasgemisch befinden kann. Nachteilig an hydraulischen Rührwerken ist allerdings, dass die zum Betrieb des Rührgeräts benötigte Energie über Hydraulikleitungen von außen zugeführt werden muss. Demgegenüber ist es einfacher, ein Rührgerät mit einem elektrischen Antriebsmotor einzusetzen. Dort muss zwar auch eine entsprechende Dichtung vorgesehen sein, aber es kann auf hydraulische Zuleitungen verzichtet werden. Nachteilig an dem Einsatz eines Elektromotors ist auch, dass typische Asynchronmotoren ein relativ geringes Drehmoment aufweisen und deshalb zusammen mit einem Getriebe verwendet werden. Dadurch steigt der Aufwand und der Wirkungsgrad sinkt.

Die DE 38 187 27 A1 offenbart einen Laboraufbau mit einem vertikalen Antrieb für ein Rührwerk des kleinen Züchtungsgefäßes. Der Rotor und der Stator sind beide scheibenförmig ausgebildet, axial zueinander beabstandet und außerhalb des Züchtungsgefäßes gekapselt angeordnet. Die scheibenförmige Ausbildung von Rotor und Stator und das stabförmige Rührwerk limitieren die Steifigkeit und die maximal übertragbaren Kräfte. Außerdem ist nachteilig, dass zum Wechsel des Rührwerks der Laboraufbau komplett demontiert werden muss.

Die DE 20 2005 005 165 U1 offenbart ein tragbares Hydraulikaggregat mit einem permanenterregten Synchronmotor und mehreren Tauchpumpen. Das Reservoir umschließt den Stator und den Rotor zur Kühlung direkt. Die Bauform eignet sich vor allem nicht für das hochviskose Substrat in Fermentern. Es kann zu einer Verstopfung, hohen Temperaturen oder sogar Explosionen kommen.

Es ist deshalb die Aufgabe der vorliegenden Erfindung, eine verbesserte elektrische angetriebene Rühreinrichtung für einen Fermenter einer Biogasanlage sowie einen Fermenter mit einer solchen Rühreinrichtung zur Verfügung zu stellen, welche einen hohen Wirkungsgrad aufweist und für die Übertragung hoher Drehmomente bei kleinen Drehzahlen geeignet ist.

Diese Aufgabe wird gelöst durch eine Rühreinrichtung mit den Merkmalen des Anspruchs 1 und durch einen Fermenter mit den Merkmalen des Anspruchs 11. Bevorzugte Weiterbildungen der Erfindung sind Gegenstand der Unteransprüche. Weitere Vorteile und Merkmale der vorliegenden Erfindung ergeben sich aus der allgemeinen Beschreibung und aus der Beschreibung des Ausführungsbeispiels.

Eine erfindungsgemäße Rühreinrichtung ist für einen Fermenter einer Biogasanlage vorgesehen. Die Rühreinrichtung weist ein Gehäuse und eine Antriebseinrichtung zum drehbaren Antreiben wenigstens eines Rührflügels auf. Die Antriebseinrichtung umfasst eine Antriebswelle und einen elektrischen Antriebsmotor. Der Antriebsmotor ist abgedichtet in dem Gehäuse aufgenommen. Der Antriebsmotor weist einen äußeren hohlen Stator und einen zentrisch darin aufgenommenen wenigstens teilweise hohl ausgebildeten rotierbaren Rotor auf. Der Rotor ist drehbar an dem Gehäuse gelagert und weist eine Kopplungseinrichtung zur drehfesten Kopplung mit der Antriebswelle auf, um mittels der Antriebswelle den wenigstens einen Rührflügel anzutreiben. Der Stator ist mit einer Mehrzahl von elektrischen Wicklungen und der Rotor mit einer Mehrzahl an Permanentmagneten ausgerüstet. Der Antriebsmotor ist als Direktantrieb ausgebildet und die Antriebswelle ist getriebelos mit dem Antriebsmotor verbunden. Ein Innendurchmesser des hohlen Teils des Rotors ist wenigstens doppelt so groß ist wie ein Außendurchmesser der Antriebswelle.

Die erfindungsgemäße Rühreinrichtung hat viele Vorteile. Ein erheblicher Vorteil der erfindungsgemäßen Rühreinrichtung besteht darin, dass ein elektrischer Antriebsmotor mit einem äußeren hohlen Stator verwendet wird. Dabei wird innerhalb des hohlen Stators ein wenigstens teilweise hohl ausgebildeter rotierbarer Rotor angeordnet. Dadurch wird der Antriebsmotor mit größeren äußeren Abmessungen vorgesehen, sodass der hohl ausgebildete Stator mit dem daran zentrisch aufgenommenen und hohl ausgebildeten rotierbaren Rotor für die Übertragung hoher Drehmomente ausgebildet ist. Dadurch kann der Nachteil aus dem Stand der Technik überwunden werden und es ist möglich, den Antriebsmotor getriebelos mit der Antriebswelle zu koppeln.

Die Antriebseinrichtung ist bei Einsätzen in einem Fermenter einer Biogasanlage flüssigkeitsdicht und gasdicht ausgebildet. Die Rühreinrichtung ist dazu ausgebildet und vorgesehen, in das Substrat im Inneren des Fermenters einzutauchen. Die Rühreinrichtung kann eine Mehrzahl von Rührflügeln aufweisen, wobei die Zahl der Rührflügel vorzugsweise zwei, drei, vier oder fünf oder mehr beträgt.

Erfindungsgemäß ist der Stator mit einer Mehrzahl von elektrischen Wicklungen und der Rotor ist mit einer Mehrzahl an Permanentmagneten ausgerüstet. Beispielsweise kann der Stator mit 40-80 Zahnspulenwicklungen und der Rotor mit 40-100 Oberflächenmagneten versehen sein. In einer bevorzugten Ausgestaltung werden 60 Wicklungen und 70 Oberflächenmagnete eingesetzt. Die hohe Zahl von mehr als zehn und insbesondere mehr als 20 elektrischen Wicklungen und Permanentmagneten führen zu einem guten Ansprechverhalten und zu einem hohen Drehmoment, welches auch bei geringen Drehzahlen zuverlässig zur Verfügung steht. Möglich sind auch z.B. Pole, 35 Polpaare und/oder 280 Magnete oder mehr.

Erfindungsgemäß ist der Antriebsmotor als Direktantrieb ausgebildet und die Antriebswelle ist getriebelos mit dem Antriebsmotor gekoppelt. Insbesondere kann auch die Fügelnabe getriebelos mit dem Antriebsmotor gekoppelt sein. Eine solche Ausgestaltung ist sehr vorteilhaft, da Übertragungsverluste durch das Getriebe vermieden werden können. Außerdem stellt ein Getriebe ein Verschleißteil dar, welches nur eine begrenzte Lebensdauer aufweist. Durch den Aufbau, die Ansteuerung und die Maße dieser Weiterbildung der erfindungsgemäßen Rühreinrichtung werden ein hoher Wirkungsgrad und eine hohe Zuverlässigkeit erreicht.

In einer bevorzugten Weiterbildung umfasst die Kopplungseinrichtung des Rotors einen Zahnflansch, der eine Innenverzahnung zur drehfesten Aufnahme der mit einer Außenverzahnung ausgerüsteten Antriebswelle aufweist. Ein solcher Zahnflansch erlaubt eine zuverlässige und schnelle und einfache Kopplung der Antriebswelle mit dem Antriebsmotor.

Vorzugsweise umfasst die Antriebseinrichtung eine Vorsatzeinrichtung mit wenigstens einer Lagereinrichtung zur drehbaren Lagerung der Antriebswelle, wobei die Vorsatzeinrichtung mit dem Gehäuse abnehmbar verbunden ist. Insbesondere durch die Zusammenwirkung des Zahnflansches und einer Vorsatzeinrichtung mit einer Lagereinrichtung zur drehbaren Lagerung der Antriebswelle wird ein einfacher und dennoch zuverlässiger Aufbau der Antriebseinrichtung der Rühreinrichtung ermöglicht und gewährleistet. Auch die Dichtung des Antriebsmotors gegen Flüssigkeit und Gas wird konstruktiv einfacher. Die Antriebswelle tritt nur von einer Seite in das Gehäuse des Antriebsmotors ein, sodass die Antriebswelle nur an einer Stelle abgedichtet werden muss. In diesen Ausgestaltungen ist es möglich, dass die Vorsatzeinrichtung an das Gehäuse der Antriebseinrichtung bzw. des Antriebsmotors angeschraubt oder sonstwie damit befestigt ist. In bevorzugten Ausgestaltungen ist eine Flügelnabe drehfest auf der Antriebswelle angeordnet und der wenigstens eine Rührflügel ist an der Flügelnabe angebracht.

Die Antriebswelle ist in allen Ausgestaltungen in der Regel fest mit der Flügelnabe verbunden. Bevorzugt ist eine Ausgestaltung, bei der wenigstens ein radialer Mitnehmer an der Antriebswelle oder an der Flügelnabe in eine entsprechende Nut oder in eine entsprechende Aussparung der Flügelnabe eingreift, um eine drehfeste Verbindung herzustellen.

In einer bevorzugten Weiterbildung ist die Flügelnabe mittels einer Fixiereinheit am vorderen Ende der Antriebswelle in axialer Richtung fixiert. Die Fixiereinheit kann in einfachen Ausgestaltungen als eine Art Deckel oder dergleichen ausgebildet sein. Möglich ist es auch, dass die Fixiereinheit durch einen ausreichend starken Stift oder dergleichen gebildet wird. In entsprechender Weise ist vorzugsweise ein hinterer Anschlag an der Antriebswelle für die Flügelnabe vorgesehen.

In besonders bevorzugten Weiterbildungen ist ein Außendurchmesser der Flügelnabe wenigstens doppelt so groß und insbesondere wenigstens dreimal so groß wie ein Außendurchmesser der Antriebswelle. Dabei dabei wird unter dem Außendurchmesser der Flügelnabe der Durchmesser ohne die Rührflügel bzw. mit abmontierten Rührflügeln verstanden.

Erfindungsgemäß ist ein Innendurchmesser des hohlen Teils des Rotors wenigstens doppelt so groß wie ein Außendurchmesser der Antriebswelle. Dadurch wird ein besonders großer Durchmesser des krafteinbringenden Teils des Antriebsmotors bewirkt, wodurch besonders große Drehmomente auch bei kleinen Drehzahlen übertragen werden können.

Besonders bevorzugt ist deshalb ein Außendurchmesser des Rotors wenigstens dreimal und insbesondere wenigstens viermal so groß wie ein Außendurchmesser der Antriebswelle. Dadurch wird ein großdimensionierter Antriebsmotor definiert, der getriebelos arbeiten kann, sodass die Kosten und das Bauvolumen eines Getriebes eingespart werden können. Außerdem steigt der Wirkungsgrad, da ein zusätzliches und mit Übertragungsverlusten behaftetes Bauteil eingespart werden kann.

Vorzugsweise ist der Außendurchmesser des Rotors größer als ein Außendurchmesser der Flügelnabe.

Vorteilhaft bildet die Außenseite des Stators eine Außenfläche des Gehäuses.

In bevorzugten Ausgestaltungen steht die Antriebswelle von einer Vorderseite des Gehäuses ab. Vorteilhafterweise ist auf der Rückseite des Gehäuses eine Konsolenaufnahme zur Befestigung an einer Konsole angeordnet. Mit der Konsolenaufnahme wird das Gehäuse an einer Konsole befestigt bzw. angeordnet, wobei die Konsole insbesondere höhenverstellbar an einer Stützeinheit in Form beispielsweise einer Stützstange oder dergleichen angeordnet ist. Vorzugsweise ist die Stützstange drehbar angeordnet, um insgesamt unterschiedliche Höhen und Ausrichtungen des Rührgeräts in dem Fermenter zu ermöglichen.

Vorzugsweise erstreckt sich die Antriebswelle von dem Zahnflansch durch eine Wellenöffnung in einem Stirndeckel aus dem Gehäuse heraus nach außen. Dabei ist insbesondere an der Wellenöffnung wenigstens eine Wellendichtung zwischen dem Stirndeckel und der Antriebswelle angeordnet. Dadurch wird schon an der Eintrittsstelle der Antriebswelle in das Gehäuse hinein eine zuverlässige Dichtung erzielt. Dadurch wird die Erreichung des Ziels, einen absolut dichten Antriebsmotor zur Verfügung zu stellen, erheblich erleichtert.

In allen Ausgestaltungen können mehrere Rührflügel vorgesehen sein.

Der erfindungsgemäße Fermenter einer Biogasanlage weist einen wenigstens teilweise mit Substrat füllbaren Fermenterinnenraum auf. In dem Fermenterinnenraum ist wenigstens eine über eine Steuereinrichtung gesteuerte Rühreinrichtung angeordnet. Die Rühreinrichtung weist ein Gehäuse, wenigstens einen Rührflügel und eine Antriebseinrichtung zum drehbaren Antreiben des wenigstens einen Rührflügels auf. Die Antriebseinrichtung umfasst eine Antriebswelle und einen elektrischen Antriebsmotor, wobei der Antriebsmotor abgedichtet in dem Gehäuse aufgenommen ist. Der Antriebsmotor umfasst einen äußeren hohlen Stator und einen zentrisch darin aufgenommenen und wenigstens teilweise hohl ausgebildeten rotierbaren Rotor.

Der Rotor ist drehbar an dem Gehäuse gelagert und weist eine Kopplungseinrichtung zur drehfesten Kopplung mit der Antriebswelle auf, um mittels der Antriebswelle den wenigstens einen Rührflügel anzutreiben. Der Stator ist mit einer Mehrzahl von elektrischen Wicklungen und der Rotor mit einer Mehrzahl an Permanentmagneten ausgerüstet. Der Antriebsmotor ist als Direktantrieb ausgebildet und die Antriebswelle ist getriebelos mit dem Antriebsmotor verbunden. Ein Innendurchmesser des hohlen Teils des Rotors ist wenigstens doppelt so groß ist wie ein Außendurchmesser der Antriebswelle.

Auch der erfindungsgemäße Fermenter hat viele Vorteile, da durch einen oder mehrere in dem Fermenterinnenraum angeordnete Rühreinrichtungen ein zuverlässiger und effizienter Betrieb ermöglicht wird.

Vorzugsweise ist der Rotor innenliegend vorgesehen und der Stator umgibt den Rotor. Der Rotor ist dabei insbesondere als Hohlwelle ausgebildet.

Besonders bevorzugt wird die Antriebseinrichtung über einen Frequenzumrichter angesteuert. Möglich ist auch der Einsatz von zwei oder mehr Frequenzumrichtern.

Vorzugsweise ist der Antriebsmotor geeignet, bei einer Drehzahl von 1 Umdrehung/s bzw. bei einer Drehzahl von 60 Umdrehungen/min ein Drehmoment von wenigstens 250 Nm und insbesondere von wenigstens 300 Nm aufzubringen. Typisch ist ein Drehmoment von wenigstens 500 Nm bei einer Drehzahl von 120 Umdrehungen/min. Das Drehmoment kann 1000 Nm überschreiten.

Der Antriebsmotor ist in allen Ausgestaltungen insbesondere für Drehzahlen von bis zu 150 und insbesondere bis zu 200 Umdrehungen/min oder mehr ausgebildet. Insbesondere ist der Antriebsmotor für Drehzahlen zwischen 30 und 180 Umdrehungen/min vorgesehen.

120 Umdrehungen/min. Das Drehmoment kann 1000 Nm überschreiten.

Der Antriebsmotor ist in allen Ausgestaltungen insbesondere für Drehzahlen von bis zu 150 und insbesondere bis zu 200 Umdrehungen/min oder mehr ausgebildet. Insbesondere ist der Antriebsmotor für Drehzahlen zwischen 30 und 180 Umdrehungen/min vorgesehen.

In allen Ausgestaltungen ist die Rühreinrichtung insbesondere automatisch höhenverstellbar und/oder seitenverstellbar.

Weitere Vorteile und Anwendungsmöglichkeiten der vorliegenden Erfindung ergeben sich aus dem Ausführungsbeispiel, welches im Folgenden mit Bezug auf die beiliegenden Figuren erläutert wird.

In den Figuren zeigen:
- Figur 1: einen schematischen Seitenquerschnitt durch einen Fermenter;
- Figur 2: eine perspektivische Ansicht einer Rühreinrichtung für den Fermenter gemäß Figur 1;
- Figur 3: eine geschnittene schematische Seitenansicht der Rühreinrichtung nach Figur 2;
- Figur 4: eine geschnittene schematische Seitenansicht der Rühreinrichtung nach Figur 3 ohne die Rührflügel;
- Figur 5: den Antriebsmotor der Rühreinrichtung nach Figur 4 im Schnitt;
- Figur 6: eine Vorderansicht der Rühreinrichtung gemäß Figur 5;

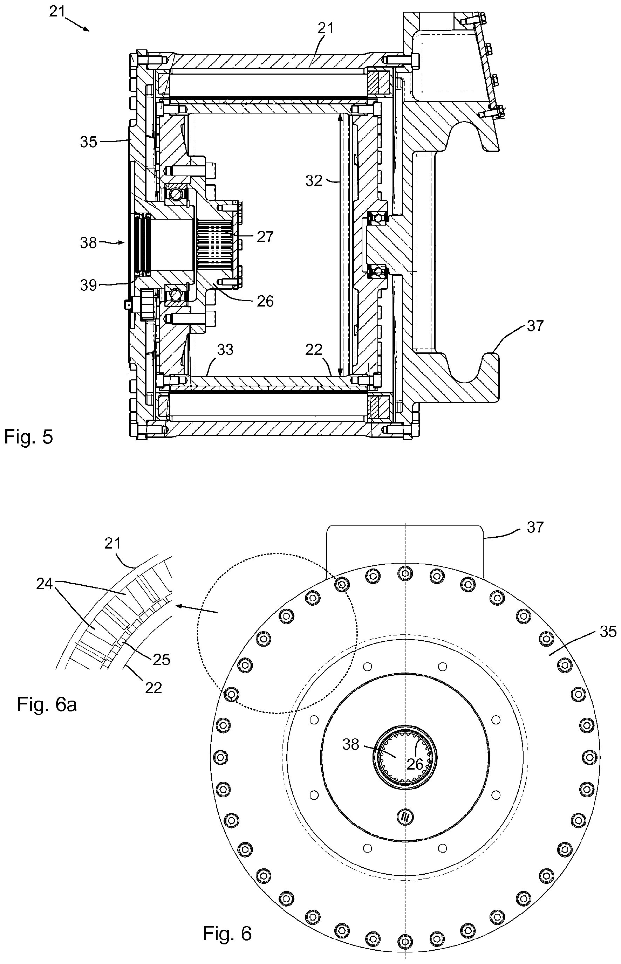

- Figur 6: ein vergrößertes Querschnittsdetail aus Figur 6;

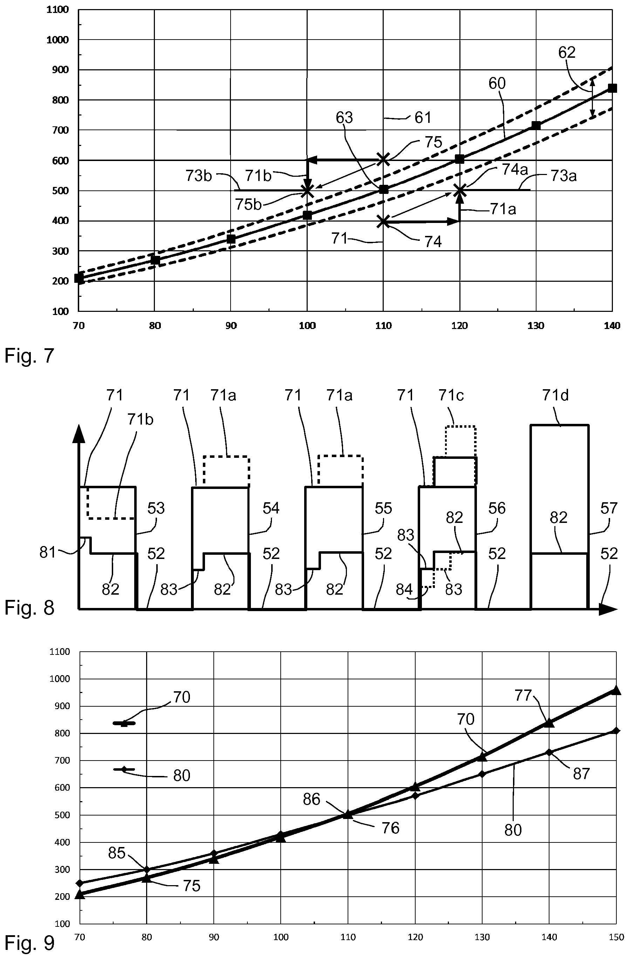

- Figur 7: eine Soll-Lastkurve für ein bestimmtes Substrat;
- Figur 8: die Drehzahl der Rühreinrichtung über der Zeit;
- Figur 9: eine Darstellung zweier aufgenommener unterschiedlicher Lastkurven.

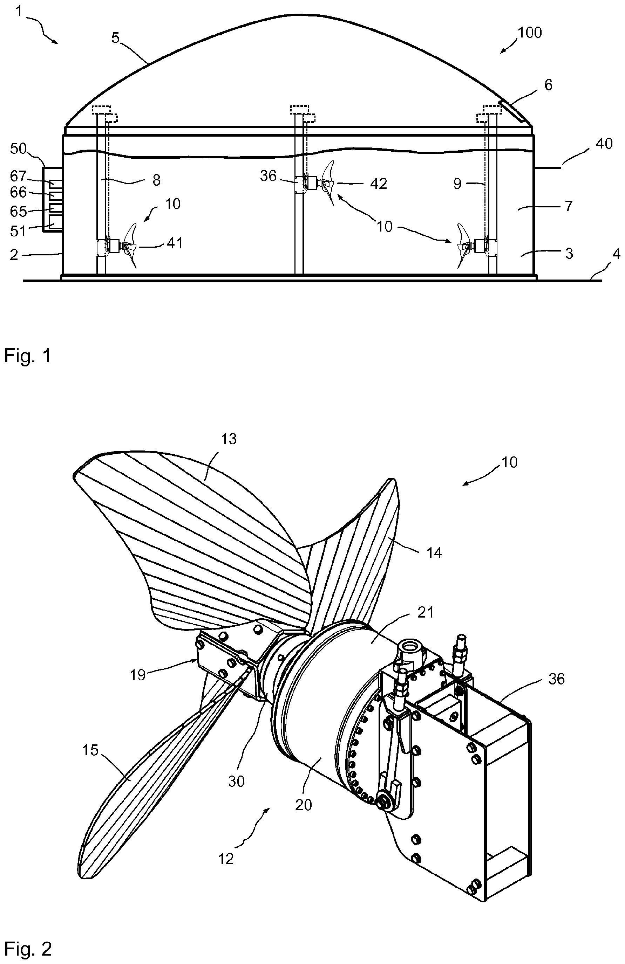

Mit Bezug auf die Figuren wird nun ein Ausführungsbeispiel erläutert. Dabei zeigt Figur 1 in einer stark schematischen Seitenansicht einen Fermenter 1 einer Biogasanlage 100.

Der Fermenter 1 weist vorzugsweise einen etwa kreisförmigen Querschnitt auf und verfügt über eine hier ringsum umlaufende Fermenterwandung 2 aus zum Beispiel Beton oder Stahl. Das Fermenterdach 5 kann ebenso wie der Boden als flaches Stahl- oder Betondach ausgeführt sein. Hier wird das Fermenterdach 5 durch ein insbesondere flexibles Material gebildet und erstreckt sich von der Wandung aus nach oben, sodass sich eine gewölbte Struktur des Behälterdaches 5 ergibt. Der Neigungswinkel des Fermenterdaches 5 hängt von den konkreten Bedingungen ab und kann 15° oder mehr und insbesondere auch 30° oder 45° erreichen oder überschreiten. Vorzugsweise ist das Fermenterdach 5 wenigstens teilweise und insbesondere vollständig entfernbar, um den Fermenterinnenraum 3 zugänglich zu machen. In dem Fermenterinnenraum 3 ist im Betrieb ein Substrat 7 vorgesehen.

In dem Fermenterdach 5 kann wenigstens eine Serviceöffnung 6 vorgesehen sein, um beispielsweise ein in dem Fermenterinnenraum 3 angeordnetes Rührgerät 10 zu warten. Eine Plattform 40 kann kann beispielsweise an der Außenseite der Fermenterwandung 2 befestigt sein, damit eine Bedienperson sich darauf stellen kann.

Figur 2 zeigt eine eine schematische perspektivische Darstellung der Rühreinrichtung 10 mit der Antriebseinrichtung 12. Die Rühreinrichtung 10 wird mittels einer Konsole 36 an der als Stützstange ausgebildeten Stützeinheit 8 höhenverstellbar aufgenommen. Das Rührgerät 10 ist zusammen mit der Stützeinheit 8 verschwenkbar und kann um 360° verdreht werden. Dadurch wird es ermöglicht, die Rührflügel 13, 14 und 15 zur Fermenterwandung 2 hin zu verschwenken und für Wartungszwecke nach oben zu verfahren, wo die Rühreinrichtung dann durch die Serviceöffnung 6 zugänglich ist.

Wie in Figur 1 dargestellt, können zwei, drei oder noch mehr Rühreinrichtungen 10 in dem Fermenterinnenraum 3 angeordnet werden, um für eine zuverlässige und ausreichende Durchmischung des Substrates 7 zu sorgen. Dabei ist es möglich, die verschiedenen Rühreinrichtungen 10 auf unterschiedlichen Höhen 41, 42 zu positionieren, um auf der Höhenposition 41 beispielsweise im unteren Bereich des Fermenters 1 umzurühren, während an der Höhenposition 42 ein oberer Bereich durchmischt wird, um Schwimmschichten aufzulösen bzw. zu vermeiden.

Neben den eingezeichneten Höhenstellungen 41 und 42 sind noch weitere Höhenstellungen möglich, insbesondere eine mittlere Höhenposition zwischen der ersten Höhenposition 41 und der zweiten Höhenstellung 42.

Vorzugsweise sind wenigstens zwei Rühreinrichtungen 10 vorgesehen, die jeweils um die Achse der Stützeinheit 8 verschwenkbar sind, um unterschiedliche Durchmischungen und Strömungsrichtungen innerhalb des Substrates 7 erzeugen zu können. Dabei können die Rühreinrichtungen 10 in die gleiche Umlaufrichtung oder schräg zueinander oder in entgegensetzte Umlaufrichtungen ausgerichtet sein. Der Einsatz ist auf der gleichen Höhe oder in unterschiedlichen Höhenpositionen möglich. Jede Rühreinrichtung 10 wird entweder durch eine eigene Steuereinrichtung 50 oder durch eine den Rührgeräten bzw. Rühreinrichtungen 10 gemeinsame Steuereinrichtung 50 gesteuert. Die Ansteuerung erfolgt hier über einen Frequenzumrichter 51.

Wie Figur 2 zeigt, weist eine Rühreinrichtung hier im Ausführungsbeispiel drei Rührflügel 13, 14 und 15 auf, die an einer Flügelnabe 19 angebracht sind. Die Flügelnabe 19 ist wiederum an der in Figur 2 nicht sichtbaren Antriebswelle drehfest befestigt.

Die Antriebseinrichtung 12 umfasst den Antriebsmotor 20 und die Vorsatzeinrichtung 30, die an dem Gehäuse des Antriebsmotors 20 befestigt ist. Der Antriebsmotor 20 weist einen großen Durchmesser auf, der durch den Außendurchmesser des Stators 21 wesentlich bestimmt wird. Der Stator 21 bildet mit seiner Außenseite einen Teil des Gehäuses des Antriebsmotors 20.

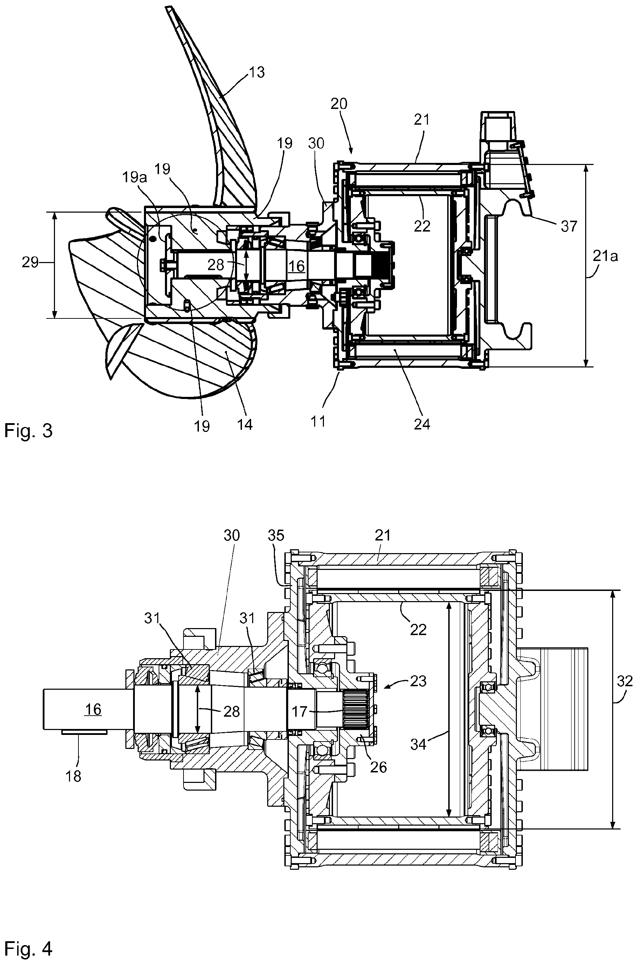

Figur 3 zeigt eine geschnittene schematische Seitenansicht der Rühreinrichtung 10, wobei hier die hinteren Rührflügel 13 und 14 zu sehen sind.

Erkennbar ist, dass an dem Antriebsmotor 20 eine Vorsatzeinrichtung 30 angebracht ist. Die Vorsatzeinrichtung dient zur Lagerung und Führung der Antriebswelle 16. Auf der Antriebswelle 16 ist die Flügelnabe 19 befestigt, an der wiederum die einzelnen Rührflügel 13 bis 15 befestigt sind. Die Konsolenaufnahme 37 dient zur Befestigung an der Konsole 36. Das Gehäuse 11 wird zum Teil durch den Stator 21 gebildet, der einen Außendurchmesser 21a aufweist. Im Inneren des Stators 21 ist hier ein hohl ausgebildeter Rotor 22 angeordnet. Der Stator 21 weist einen Außendurchmesser 21a auf. Die Antriebswelle 16 weist einen Außendurchmesser 28 auf. Der Außendurchmesser 21a des Stators ist mehrfach größer als der Außendurchmesser 28 der Antriebswelle 16. Dadurch wird ein besonders starkes Drehmoment des Antriebsmotors 20 erzielt. Hier ist auch ein Außendurchmesser 29 der Flügelnabe 19 erheblich kleiner als ein Außendurchmesser 21a des Stators.

Figur 4 zeigt einen detaillierteren Querschnitt des Antriebsmotors 20 mit der daran anmontierten Vorsatzeinrichtung 30. Die Vorsatzeinrichtung 30 ist an dem Stirndeckel 35 des Gehäuses 11 des Antriebsmotors 20 angebracht. In dem Inneren der Vorsatzeinrichtung 30 sind Lagereinrichtungen 31 zur Lagerung der Antriebswelle 16 angeordnet. Die Antriebswelle 16 verfügt über einen Mitnehmer 18, der radial nach außen absteht und in eine entsprechende Nut oder dergleichen in der Flügelnabe eintaucht.

Die Kopplung der Antriebswelle 16 mit dem Rotor 22 erfolgt über eine Kopplungseinrichtung 23, die hier als Zahnflansch 26 ausgebildet ist und die den Rotor 22 nach außen hin abdichtet. Der Zahnflansch 26 weist eine Innenverzahnung 27 auf, die im montierten Zustand, wie in Figur 4 dargestellt, im Eingriff mit der Außenverzahnung 17 der Antriebswelle 16 steht. Durch die Vorsatzeinrichtung 30 wird eine einfache und leichte Montage und ein einfacher Austausch ermöglicht. Im Bedarfsfalle wird die Vorsatzeinrichtung zusammen mit der Antriebswelle der Antriebseinrichtung 12 entnommen und kann durch eine neue ausgetauscht werden.

In Figur 4 ist der Innendurchmesser 32 des Rotors 22 21 eingezeichnet, Der Außendurchmesser 34 des Rotors 22 entspricht dem Innendurchmesser des Stators 21.

Der Innendurchmesser 32 beträgt ein Vielfaches des Außendurchmessers 28 der Antriebswelle 16, sodass von dem Antriebsmotor 20 hohe Drehmomente übertragbar sind.

Figur 5 zeigt den Antriebsmotor der Rühreinrichtung ohne Vorsatzeinrichtung im Schnitt. Dabei ist an der Rückseite die Konsolenaufnahme 37 und an der Vorderseite der Stirndeckel 35 zu sehen. In dem Stirndeckel 35 ist eine Wellenöffnung 38 zur Aufnahme der Antriebswelle 16 vorgesehen. An der Wellenöffnung 38 ist wenigstens eine Wellendichtung 39 angeordnet, um den Antriebsmotor nach innen hin abzudichten.

Nach dem Einführen der Antriebswelle 16 in die Wellenöffnung 38 greift die Außenverzahnung 17 der Antriebswelle 16 in die Innenverzahnung 27 des Zahnflansch 26 der Kopplungseinrichtung 23 ein. Es liegt eine drehfeste Kopplung zwischen der Antriebswelle und dem Antriebsmotor 20 vor. Durch den Aufbau mit einem hohlen Rotor 22 mit einem großen Innendurchmesser 32 wird ein leichter Aufbau mit einer hohen Drehmomentübertragbarkeit ermöglicht. Außerdem kann die Antriebswelle 16 ohne Öffnung des Antriebsmotors 20 ausgetauscht werden.

Figuren 6 und 6a zeigen eine Frontansicht bzw. eine vergrößerte geschnittene Frontansicht des Antriebsmotors 20 ohne Vorsatzeinrichtung 30. Im Hintergrund ist die Konsolenaufnahme 37 zu sehen, während vorn der Stirndeckel 35 mit der darin vorgesehenen Wellenöffnung 38 zu erkennen ist. Erkennbar ist der Zahnflansch 26 mit der Innenverzahnung 27.

Figur 6a zeigt eine vergrößerte geschnittene Darstellung eines Teils des Antriebsmotors 20, in der ein Winkelsegment des Stators 21 und des Rotors 22 erkennbar sind. An dem Stator 21 sind mehrere Wicklungen 24 vorgesehen, während an dem Rotor 22 Permanentmagnete 25 angeordnet sind. Vorzugsweise ist die Zahl der Wicklungen größer als die Zahl der Permanentmagnete und besonders bevorzugt beträgt die Anzahl der Permanentmagnete und der Wicklungen jeweils größer 30 und besonders bevorzugt größer 50. Durch die hohe Anzahl von Wicklungen und Permanentmagneten wird eine genaue Steuerung und es wird ein hohes Drehmoment ermöglicht. In bevorzugten Ausgestaltungen ist der Antriebsmotor 20 als Torquemotor ausgeführt. In einer bevorzugten Ausgestaltung sind z.B. mindestens 70 Pole, 35 Polpaare und/oder 280 Magnete vorgesehen.

Figur 7 zeigt eine Soll-Lastkurve 60 für ein bestimmtes Substrat 7 und einen sich um die Soll-Lastkurve 60 prozentual herum erstreckenden Toleranzbereich 62. Möglich ist es, dass sich der Toleranzbereich 62 um einen festen Wert um die Soll-Lastkurve herum erstreckt. Möglich ist auch ein relativer Prozentsatz von zum Beispiel 5 % oder 10 % Abweichung nach oben und nach unten.

In Figur 7 ist das Drehmoment in Newtonmetern über der Drehzahl in Umdrehungen pro Minute für einen konkreten Fall aufgetragen. Diese Soll-Lastkurve kann empirisch ermittelt werden und beispielsweise für ein bestimmtes Substrat mit einer bestimmten Zusammensetzung etc. gelten. Diese Soll-Lastkurve 60 wird vorgegeben, um über die Steuereinrichtung 50 die Rühreinrichtung 10 bzw. die Rühreinrichtungen 10 entsprechend gezielt steuern zu können.

Wie hier erkennbar, steigt das Drehmoment mit steigender Drehzahl an.

Grundsätzlich funktioniert die Steuerung bei dem Fermenter 1 derart, dass zunächst bei Start der Anlage eine Soll-Lastkurve 60 vorgegeben oder aus einer Speichereinrichtung abgerufen wird. Im Anschluss wird eine Soll-Drehzahl 61 durch die Steuereinrichtung 50 vorgegeben. Die Steuereinrichtung 50 betreibt die Rühreinrichtung 10 mit einer Ist-Drehzahl, die der vorgegebenen Soll-Drehzahl wenigstens etwa entspricht. Nach Erreichen der Ist-Drehzahl 71 wird an dem Betriebspunkt 74,75 ein Ist-Messwert aufgenommen, der für das Drehmoment oder die Leistung der Rühreinrichtung 10 bei der Ist-Drehzahl 71 charakteristisch ist. Beispielsweise kann über einen Dehnungsmessstreifen oder dergleichen auf der Antriebswelle oder in oder an dem Rotor ein Messwert aufgenommen werden, der charakteristisch für das anliegende Drehmoment ist. Möglich und bevorzugt ist es aber auch, einen solchen Messwert 73a,73b direkt aus der elektrischen Leistungsaufnahme der Rühreinrichtung 10 abzuleiten. Der Messwert kann direkt als Kennwert verwendet werden oder der Kennwert wird aus dem Messwert berchnet.

Aus dem Messwert wird ein Ist-Kennwert 74,75 abgeleitet. Dieser Ist-Kennwert wird mit dem sich aus der Soll-Lastkurve 60 bei der vorgegebenen Soll-Drehzahl 61 ergebenden Soll-Kennwert 63 für das Substrat verglichen.

Stellt die Steuereinrichtung fest, dass das auftretende Drehmoment bei der Ist-Drehzahl 71 außerhalb des Toleranzbereichs 62 bei der Soll-Drehzahl 61 liegt, so wird entweder die Ist-Drehzahl um ein vorbestimmtes Maß erhöht oder aber abgesenkt, je nachdem, ob das Ist-Drehmoment größer oder kleiner als der Soll-Kennwert 63 ist.

Im dargestellten Ausführungsbeispiel wird die Ist-Drehzahl 71 jeweils um 10 Umdrehungen/min erhöht oder reduziert. Möglich ist es aber auch, dass die Drehzahl in kleineren Schritten oder aber prozentual in Abhängigkeit von der Soll-Drehzahl 61 verändert wird.

Nach der Erhöhung der Ist-Drehzahl auf den Wert 71a steigt das steigt der Ist-Messwert und somit der Ist-Kennwert auf den Wert 73a, der hier im gewählten Ausführungsbeispiel innerhalb des Toleranzbereichs 62 um die Soll-Lastkurve 60 bei der Soll-Drehzahl 61 liegt. Durch die Erhöhung der Drehzahl hat sich das Drehmoment soweit erhöht, dass das Drehmoment nun im gewünschten Bereich liegt.

Tritt der umgekehrte Fall auf, d.h., dass das bei der Soll-Drehzahl 61 ein Drehmoment anliegt, welches oberhalb des Toleranzbereichs 62 der Soll-Lastkurve 60 liegt, so wird die Ist-Drehzahl 71 auf die Ist-Drehzahl 71b reduziert. Durch die geringere Drehzahl bedingt, nimmt auch das erforderliche Drehmoment ab, so dass der Ist-Kennwert 73b bei der verringerten Ist-Drehzahl 71b nun innerhalb des Toleranzbereichs 62 der Soll-Lastkurve 60 bei der Soll-Drehzahl 61 liegt.

Damit ist in beiden Fällen - also bei einer Überschreitung des Drehmomente nach oben und nach unten - jeweils dafür gesorgt, dass das Ist-Drehmoment sicher in dem gewünschten Bereich liegt. Im Anschluss wird für den Rest eines Rührzyklus die Rühreinrichtung mit der so ermittelten Ist-Drehzahl 71, 71a, oder 71b weiterbetrieben.

Falls die Erhöhung bzw. Absenkung des Ist-Drehmomente in einem Schritt nicht ausreichen sollte, wird die oben beschriebene Schleife iterativ durchgelaufen, bis das Ist-Drehmoment in dem gewünschten Zielbereich liegt.

Das bedeutet, dass bei dem Verfahrensablauf zunächst eine Soll-Lastkurve 60 in der Steuereinrichtung 50 hinterlegt wird bzw. aus einer Speichereinrichtung oder aus der Steuereinrichtung 50 eine Soll-Lastkurve 60 abgerufen wird.

Die Steuereinrichtung 50 gibt bei Beginn eines jeden Rührzyklus eine Soll-Drehzahl vor, zunächst die Soll-Drehzahl 61. Die Steuereinrichtung 50 steuert die Rühreinrichtung 10 entsprechend an, sodass die Rühreinrichtung 10 eine Ist-Drehzahl 71 erreicht, die im Rahmen der Regelgenauigkeit der vorgegebenen Soll-Drehzahl 61 entspricht. Es ergibt sich - je nach Eigenschaften des Substrates ein Betiebspunkt74 oder ein Betriebspunkt 75.

Anschließend erfasst die Steuereinrichtung einen Ist-Messwert 81 (vgl. Fig. 8), der für das Drehmoment der Rühreinrichtung 10 bei der Ist-Drehzahl 71 charakteristisch ist. Der Messwert 81 ist insbesondere die elektrische Leistungsaufnahme der Rühreinrichtung bei der Ist-Drehzahl 71,kann aber auch direkt das Drehmoment sein

Aus dem Ist-Messwert leitet die Steuereinrichtung 50 unter Berücksichtigung der Gerätefaktoren, der auftretenden Verluste etc. einen Ist-Kennwert für das aufgebrachte Drehmoment ab. Der Ist-Kennwert kann auch der abgegebenen Leistung bei der Ist-Drehzahl entsprechen, da sich aus der Leistung das Drehmoment bei bekannter Drehzahl berechnen lässt. Der Ist-Kennwert kann in einfachen Fällen dem Ist-Messwert entsprechen.

Die Steuereinrichtung 50 vergleicht im Anschluss den abgeleiteten Ist-Kennwert 81 mit dem sich aus der Soll-Lastkurve 60 bei der vorgegebenen Soll-Drehzahl 61 ergebenden Soll-Kennwert 63.

In Abhängigkeit von dem Ergebnis des Vergleichs steuert die Steuereinrichtung 50 die Rühreinrichtung 10.

Dabei ermittelt die Steuereinrichtung 50 insbesondere, ob der Ist-Kennwert innerhalb eines vorgegebenen Toleranzbereichs 62 um die Soll-Lastkurve 60 bei der Soll-Drehzahl 61 liegt.

Danach wird im Fall des Betriebspunktes 74, wenn nämlich der Ist-Kennwert unterhalb des Soll-Kennwerts 63 und außerhalb des Toleranzbereichs 62 liegt, die Ist-Drehzahl 71 der Rühreinrichtung 10 um ein vorbestimmtes Maß (hier 10 Umdrehungen/min) erhöht und es ergibt sich ein neuer Betriebspunkt 74a bei der neuen Ist-Drehzahl 71a mit einem Ist-Drehmoment 73a bzw. mit einem neuen Ist-Kennwert 73a.

Dann liegt der neue Betriebspunkt 74a innerhalb des vorgegebenen Toleranzbereichs 62 der Soll-Lastkurve 60 bei der Soll-Drehzahl 61 und der Rührzyklus wird mit dieser Drehzahl weiter betrieben.

Im Fall des Betriebspunktes 75, wenn nämlich der zugehörige Ist-Kennwert oberhalb des Soll-Kennwerts 63 und außerhalb des Toleranzbereichs 62 liegt, wird die Ist-Drehzahl 71 der Rühreinrichtung 10 um ein vorbestimmtes Maß (hier 10 Umdrehungen/min) verringert und es ergibt sich ein neuer Betriebspunkt 75b bei der neuen Ist-Drehzahl 71b mit einem Ist-Drehmoment 73b bzw. mit einem neuen Ist-Kennwert 73b.

Nun liegt auch der neue Betriebspunkt 75a innerhalb des vorgegebenen Toleranzbereichs 62 der Soll-Lastkurve 60 bei der Soll-Drehzahl 61 und der Rührzyklus wird mit dieser Drehzahl 71b weiter betrieben.

Figur 8 zeigt einen schematischen zeitlichen Steuerungsablauf zur Verdeutlichung des Prinzips. Aufgetragen sind die Drehzahl 71, 71a, 71b, 71c und 71d der Rühreinrichtung 10, die Messwerte 81 bis 84 und die aus der elektrischen Leistungsaufnahme resultierenden Kennwerte bzw. Drehmomente 91 bis 94 über der Zeit. Dargestellt sind mehrere Rührzyklen 53 bis 57, die von Rührpausen 52 unterbrochen werden.

Zu Beginn des Rührzyklus 53 wird die Rühreinrichtung 10 zunächst mit einer Ist-Drehzahl 71 angesteuert bzw. betrieben, die der Soll-Drehzahl 61 entspricht. Da die gemessene elektrische Leistung 81 bzw. das das resultierende Drehmoment 91 und somit der Kennwert zunächst über dem gewünschten Soll-Kennwert liegt, wird die Ist-Drehzahl auf den Wert 71b gesenkt, sodass die elektrische Leistungsaufnahme 82 sinkt und sich ein passendes Drehmoment bzw. Kennwert 92 ergibt, der nun im gewünschten Bereich liegt. Diese Drehzahl 71b wird dann bis zum Ende des Rührzyklus 53 beibehalten.

Der Kennwert 91 und der Messwert 81 (z. B. die Leistung) können linear miteinander verknüpft sein oder über eine sonstige Formel. Möglich ist es auch, dass als Kennwerte 91 bis 94 direkt die Messwerte 81 bis 84 verwendet werden, wenn die Zuordnung eindeutig und reproduzierbar ist.

Es schließt sich an den Rührzyklus 53 ein Ruhezyklus 52 an, in welchem hier die Drehzahl der Rühreinrichtung 10 auf null abgesenkt wird.

In dem folgenden Rührzyklus 54 wird wieder mit der Ist-Drehzahl 71 gestartet, die der Soll-Drehzahl 61 entspricht. Im Rührzyklus 54 liegt die elektrische Leistungsaufnahme 83 und somit der Kennwert bzw. das Drehmoment 93 zunächst unterhalb des Sollwertes, sodass die Drehzahl auf die Ist-Drehzahl 71a erhöht wird. Danach liegen die Leistungsaufnahme 82 und das Drehmoment 92 bzw. der Ist-Kennwert 92 im gewünschten Bereich. Hier in diesem Beispiel wird aus der Leistungsaufnahme mit der Drehzahl das Drehmoment berechnet.

Nach dem nächsten Ruhezyklus findet ein Rührzyklus 55 statt, der wiederum mit der Ist-Drehzahl 71 gestartet wird, die der Soll-Drehzahl 61 entspricht. Auch in diesem Rührzyklus wird zunächst wieder eine zu geringe Leistungsaufnahme 83 und somit ein zu geringes Drehmoment 93 detektiert, sodass die Drehzahl auf die Ist-Drehzahl 71a erhöht wird, bei der das gewünschte Ist-Drehmoment 92 anliegt.

Im nächsten Rührzyklus 56 kann ein identisches Verhalten vorliegen, wie durch die durchgezogen eingezeichnete Linie dargestellt ist. Möglich ist es aber auch, dass sich die Eigenschaften des Substrates 7 geändert haben und somit eine weitere Erhöhung der Ist-Drehzahl auf einen noch höheren Wert 71c erforderlich ist, wie gestrichelt dargestellt. Bei der gestrichelt dargestellten Variante im Rührzyklus 56 muss die Ist-Drehzahl in zwei Stufen auf die Werte 71a und 71c erhöht werden, bis das gewünschte Drehmoment erreicht wird. Dabei sind der Messwert 84 und der zugehörige Kennwert 94 zunächst zu gering, steigen dann auf den Messwert 83 bzw. den Kennwert 93 und erreichen erst bei Erhöhung der Drehzahl auf den Wert 71d den Messwert 82 und das gewünschte Drehmoment bzw. den Kennwert 92.

Jedes Mal, wenn in aufeinanderfolgenden Rührzyklen die Ist-Drehzahl erhöht werden muss wird ein erster Zähler 65 (vgl. Fig. 1) erhöht, sodass der Zähler beim vierten Rührzyklus 56 auf dem Wert 3 steht. Bei Überschreitung einer vorgegebenen Schwelle 67 von z. B. 3, 5 oder 10 oder dergleichen, so wird im sich daran anschließenden Rührzyklus 57 ein neuer Startwert für die Soll-Drehzahl vorgegeben. Der neue Sollwert ist dann direkt höher als der vorhergehende. Dies ist beispielhaft in Figur 8 im letzten Rührzyklus dargestellt, bei der eine Ist-Drehzahl 71d eingestellt wird.

Wird umgekehrt die Ist-Drehzahl verringert, wird ein zweiter Zähler 66 erhöht. Überschreitet der eine (gleiche oder andere) Schwelle 67, wird auch entsprechend reagiert.

Überschreitet der erste Zähler 65 oder der zweite Zähler 66 eine Schwelle 67, da in aufeinanderfolgenden Rührzyklen jeweils in dieselbe Richtung verschoben werden musste, werden insbesondere auch Handlungsmaßnahmen ausgegeben, wie z. B. weniger oder mehr Füttern (je nach Richtung), oder es wird eine andere Rührwerksposition angefahren oder es werden längere (oder kürzere) Rührzyklen durchgeführt,

Figur 9 zeigt zwei unterschiedliche Lastkurven 70 und 80, wobei jeweils das Drehmoment in Newtonmetern (Nm) über der Drehzahl in Umdrehungen pro Minute aufgetragen ist.

Die Lastkurven 70 und 80 repräsentieren hier zwei unterschiedliche Substrate 7, wobei die Lastkurve 70 mit dem Material "Schweinegülle" aufgenommen wurde und ein niedrigviskoses Medium darstellt. Die Lastkurve 80 wurde mit dem Material Gärrest eines Fermenters aufgenommen. Dieses Substrat für die Kurve 80 stellt ein mittelviskoses Medium dar.

Hier im Ausführungsbeispiel schneiden sich die beiden Lastkurven 70 und 80 bei den Messpunkten 76 und 86,während bei dem Messpunkt 75 das erforderliche Drehmoment der Lastkurve 70 geringer ist als das entsprechende Drehmoment an dem Messpunkt 85 der Lastkurve 80.

Während hier im Ausführungsbeispiel bei den dargestellten Lastkurven 70 und 80 das zur Drehung erforderliche Drehmoment bei geringen Drehzahlen (Messpunkte 75, 85) bei der Lastkurve 80 zunächst höher ist, wird das erforderliche Drehmoment zur Drehung der Rühreinrichtung 10 bei höheren Drehzahlen bei der Lastkurve 80 geringer als bei der Lastkurve 70.

Das bedeutet, dass mit dem Fermenter 1 und der darin angeordneten Rühreinrichtung 10 es möglich ist, Lastkurven 70, 80 des darin befindlichen Substrats 7 aufzunehmen. Anhand des Drehmomentverlaufs der Lastkurven 70 bzw. 80 können auf die jeweiligen Eigenschaften und gegebenenfalls die Zusammensetzung der jeweiligen Substrate 7 Rückschlüsse gezogen werden.

Beispielsweise kann die Lastkurve 70 die Soll-Lastkurve für das verwendete Substrat 7 darstellen. Wenn nun im Laufe des Betriebes eine Lastkurve mit der Rühreinrichtung und der Steuereinrichtung 50 aufgezeichnet wird und die aufgezeichnete Lastkurve der Lastkurve 80 entspricht, so können die Unterschiede der Lastkurven 70 und 80 ausgewertet werden und es können konkrete Handlungsempfehlungen ausgegeben oder direkt veranlasst werden, um die in dem Substrat vorhandene Lastkurve an die Soll-Lastkurve anzupassen. Es kann z.B. die Zusammensetzung des zugeführten Materials verändert werden. Es ist auch möglich, die Betriebsbedingungen der Rühreinrichtungen zu ändern und beispielsweise für eine bestimmte Zeit stärker zu rühren oder gegebenenfalls schwächer. Möglich ist es auch, die Gasabnahme in Abhängigkeit von den erfassten Lastkurven zu ändern

Möglich und bevorzugt ist es auch, die Rühreinrichtung 10 in Abhängigkeit von der gewünschten Gasabnahme zu steuern. Beispielsweise kann zu bestimmten Zeiten mehr Geld für abgelieferten Strom abgerechnet werden, sodass es empfehlenswert ist zu diesen Zeitpunkten mehr Gas und insbesondere Strom zu produzieren. Dadurch kann durch einen gezielten Einsatz der Rühreinrichtungen dafür gesorgt werden, dass zu diesen Zeitpunkten oder davor (zur Speicherung) eine erhöhte Gasausgabe erfolgt.

Mit den Rühreinrichtungen, die automatisch entlang der Höhe der Stützeinheiten 8 verfahrbar angeordnet sind, ist es auch möglich, auf unterschiedlichen Höhenstellungen 41,42 etc. Lastkurven 70, 80 des im Fermenterinnenraum sich befindenden Substrates 7 aufzunehmen. Über unterschiedliche Lastkurven 70, 80 in unterschiedlichen Höhen kann auf das Vorhandensein und die Größe von Schwimmschichten und weitere Parameter des Substrates zurückgeschlossen werden. Wird beispielsweise in bestimmten Höhenschichten eine geringe Viskosität detektiert, kann dies auf das Aufschwimmen bestimmter Bestandteile oder das Absenken anderer Bestandteile hindeuten. Durch entsprechende Messungen in Schichten darüber und darunter kann so auf eine inhomogene Verteilung in dem Substrat in dem Fermenter geschlossen werden.

Durch entsprechende Ansteuerung der Rühreinrichtungen 10 (Höhe, Winkel, Intensität) kann eine vollständigere Durchmischung erzielt werden.

Gezielte Strategien zum Gasaustreiben sind möglich, so z. B. eine spiralförmige automatische Anordnung, bei der das Austreiben von unten nach oben erfolgt

Durch die Aufzeichnung von Lastkurven 70, 80 ist es auch jederzeit möglich, die in der Steuereinrichtung 50 hinterlegte Soll-Lastkurve durch eine aktuell erfasste Lastkurve zu ersetzen. Wenn der Betreiber oder der Hersteller feststellt, dass sich der Fermenter 1 im derzeitigen Betrieb so verhält, wie erwünscht, kann eine neue Soll-Lastkurve 60 erstellt und abgespeichert werden. Das kann regelmäßig oder nur auf Wunsch geschehen, beispielsweise, wenn sich die Zusammensetzung des zugeführten Substrates ändert.

Insgesamt stellt die Erfindung eine Mediums-abhängige Rührwerkstechnik zur Verfügung, bei der in Abhängigkeit von den aktuellen Bedingungen des Substrats die Steuerung automatisch erfolgt.

Der Betrieb erfolgt Energieschonend. Durch die Steuerung wird das Substrat homogenisiert. Die Sollwerte ergeben sich je nach eingesetztem Medium. Der Zustand des Medium wird lokal erfasst.

Durch die Mess- und Steuerungswerte können Handlungsmaßnahmen ausgegeben werden. Es werden Abweichungen erfasst und Korrekturmaßnahmen vorgenommen oder vorgeschlagen. Bei Störungen werden Handlungsmaßnahmen vorgeschlagen. Insgesamt sind ein vollständiges Monitoring und eine Ferndiagnose möglich. Die Wartung kann vor Ort erfolgen.

Als Rührgerät wird ein hocheffizientes getriebeloses Rührwerk mit einem verlustarmen Direktantrieb eingesetzt, welches ein über der Drehzahl konstantes Drehmoment von bis zu 1000 Nm gewährleistet. Der Drehzahlbereich erstreckt sich stufenlos von 0 - 250 Umdrehungen pro Minute.

Der Leistungsbereich des im Ausführungsbeispiel beschriebenen Geräts beträgt 4 bis 12,5 kW. Der Volumenstrom beträgt hier bis zu 153 m3/min. Durch die komfortable Höhen- und Schwenkvorrichtung wird eine sichere Positionierung in Höhe und Winkel erreicht.

Zur Steuerung kann eine multifunktionale Kontrolle der Prozessdaten wie Volumenstrom, Druck, Drehmoment, Leistung, SET Kennzahlenkurve, Kennzahlenkurvenfunktion erfolgen.

**Bezugszeichenliste:**

| | | | |
|---|---|---|---|
| 1 | Fermenter | 32 | Innendurchmesser 22 |
| 2 | Fermenterwandung | 33 | hohler Teil von 22 |
| 3 | Fermenterinnenraum | 34 | Außendurchmesser 22 |
| 4 | Horizontale | 35 | Stirndeckel |
| 5 | Fermenterdach | 36 | Konsole |
| 6 | Serviceöffnung | 37 | Konsolenaufnahme |
| 7 | Substrat | 38 | Wellenöffnung |
| 8 | Stützeinheit | 39 | Wellendichtung |
| 9 | Seil | 40 | Plattform |
| 10 | Rühreinrichtung, Rührgerät | 41 | 1. Höhenstellung |
| | | 42 | 2. Höhenstellung |
| 11 | Gehäuse | 50 | Steuereinrichtung |
| 12 | Antriebseinrichtung | 51 | Frequenzumrichter |
| 13-15 | Rührflügel | 52 | Ruhezyklus |
| 16 | Antriebswelle | 53-57 | Rührzyklus |
| 17 | Außenverzahnung | 60 | Soll-Lastkurve |
| 18 | Mitnehmer | 61 | Soll-Drehzahl |
| 19 | Flügelnabe | 62 | Toleranzbereich |
| 19a | Fixiereinheit | 63 | Soll-Kennwert |
| 20 | Antriebsmotor | 65 | erster Zähler |
| 21 | Stator | 66 | zweiter Zähler |
| 21a | Außendurchmesser 21 | 67 | Schwelle |
| 22 | Rotor | 70 | Lastkurve |
| 23 | Kopplungseinrichtung | 71 | Ist-Drehzahl |
| 24 | Wicklung | 71a,71b | Ist-Drehzahl |
| 25 | Permanentmagnet | 72a,72b | Ist-Messwert |
| 26 | Zahnflansch | 73a,73b | Ist-Kennwert |
| 27 | Innenverzahnung 26 | 74,74a | Betriebspunkt |
| 28 | Außendurchmesser 16 | 75,75a | Betriebspunkt |
| 29 | Außendurchmesser 19 | 81-84 | Messwert |
| 30 | Vorsatzeinrichtung | 91-94 | Kennwert |
| 31 | Lagereinrichtung 30 | 100 | Biogasanlage |

## Patentansprüche

1. Rühreinrichtung (10) für einen Fermenter (1) einer Biogasanlage (100) mit einem Gehäuse (11) und einer Antriebseinrichtung (12) zum drehbaren Antreiben wenigstens eines Rührflügels (13-15), wobei die Antriebseinrichtung (12) eine Antriebswelle (16) und einen elektrischen Antriebsmotor (20) umfasst, wobei der Antriebsmotor (20) abgedichtet in dem Gehäuse (11) aufgenommen ist,
**dadurch gekennzeichnet,**
**dass** der Antriebsmotor (20) einen äußeren hohlen Stator (21) und einen zentrisch darin aufgenommenen wenigstens teilweise hohl ausgebildeten rotierbaren Rotor (22) umfasst, wobei der Rotor (22) drehbar an dem Gehäuse (11) gelagert ist und eine Kopplungseinrichtung (23) zur drehfesten Kopplung mit der Antriebswelle (16) aufweist, um mittels der Antriebswelle (16) den wenigstens einen Rührflügel (13-15) anzutreiben,
und **dass** der Stator (21) mit einer Mehrzahl von elektrischen Wicklungen (24) und der Rotor (22) mit einer Mehrzahl an Permanentmagneten (25) ausgerüstet ist, und dass der Antriebsmotor (20) als Direktantrieb ausgebildet ist und die Antriebswelle getriebelos mit dem Antriebsmotor (20) verbunden ist, wobei ein Innendurchmesser (32) des hohlen Teils (33) des Rotors (22) wenigstens doppelt so groß ist wie ein Außendurchmesser (28) der Antriebswelle (16).

2. Rühreinrichtung (10) nach Anspruch 1, wobei die Kopplungseinrichtung (23) des Rotors (22) einen Zahnflansch (26) umfasst, der eine Innenverzahnung (27) zur drehfesten Aufnahme der mit einer Außenverzahnung (17) ausgerüsteten Antriebswelle (16) aufweist.

3. Rühreinrichtung (10) nach einem der vorhergehenden Ansprüche, wobei die Antriebseinrichtung (12) eine Vorsatzeinrichtung (30) mit einer Lagereinrichtung (31) zur drehbaren Lagerung der Antriebswelle (16) umfasst, wobei die Vorsatzeinrichtung (30) mit dem Gehäuse (11) abnehmbar verbunden ist.

4. Rühreinrichtung (10) nach einem der vorhergehenden Ansprüche, wobei eine Flügelnabe (19) drehfest auf der Antriebswelle (16) angeordnet und der Rührflügel (13-15) an der Flügelnabe (19) angebracht ist
und wobei die Antriebswelle (16) insbesondere wenigstens einen radialen Mitnehmer (18) zur drehfesten Verbindung mit der Flügelnabe (19) umfasst,
und/oder wobei die Flügelnabe (19) mittels einer Fixiereinheit (19a) am vorderen Ende der Antriebswelle (16) axial fixiert ist,
und/oder wobei ein Außendurchmesser (29) der Flügelnabe (19) wenigstens doppelt so groß und insbesondere wenigstens dreimal so groß ist wie der Außendurchmesser (28) der Antriebswelle (16).

5. Rühreinrichtung (10) nach einem der vorhergehenden Ansprüche, wobei ein Außendurchmesser (34) des Rotors (22) wenigstens dreimal und insbesondere wenigstens viermal so groß ist wie der Außendurchmesser (28) der Antriebswelle (16) .

6. Rühreinrichtung (10) nach einem der vorhergehenden Ansprüche ab Anspruch 4, wobei der Außendurchmesser (34) des Rotors (22) größer ist als ein Außendurchmesser (29) der Flügelnabe (19).

7. Rühreinrichtung (10) nach einem der vorhergehenden Ansprüche, wobei die Außenseite des Stators (21) eine Außenfläche des Gehäuses (11) bildet.

8. Rühreinrichtung (10) nach einem der vorhergehenden Ansprüche, wobei die Antriebswelle (16) von einer Vorderseite des Gehäuses (11) absteht und wobei auf der Rückseite des Gehäuses (11) eine Konsolenaufnahme (37) zur Befestigung an einer Konsole (36) angeordnet ist.

9. Rühreinrichtung (10) nach einem der vorhergehenden Ansprüche, wobei sich die Antriebswelle (16) von dem Zahnflansch (26) durch eine Wellenöffnung (38) in einem Stirndeckel (35) aus dem Gehäuse (11) heraus nach außen erstreckt, wobei an der Wellenöffnung (38) wenigstens eine Wellendichtung (39) zwischen dem Stirndeckel (35) und der Antriebswelle (16) angeordnet ist.

10. Rühreinrichtung (10) nach einem der vorhergehenden Ansprüche, wobei eine Mehrzahl von zwei, drei oder mehr Rührflügeln (11-13) vorgesehen ist.

11. Fermenter (1) einer Biogasanlage (100) mit einem wenigstens teilweise mit Substrat (7) füllbaren Fermenterinnenraum (3), wobei in dem Fermenterinnenraum (3) wenigstens eine über eine Steuereinrichtung (50) gesteuerte Rühreinrichtung (10) angeordnet ist, wobei die Rühreinrichtung (10) ein Gehäuse (11), wenigstens einen Rührflügel (13-15) und eine Antriebseinrichtung (12) zum drehbaren Antreiben des wenigstens einen Rührflügels (13-15) aufweist, wobei die Antriebseinrichtung (12) eine Antriebswelle (16) und einen elektrischen Antriebsmotor (20) umfasst, wobei der Antriebsmotor (20) abgedichtet in dem Gehäuse (11) aufgenommen ist,
**dadurch gekennzeichnet,**
**dass** der Antriebsmotor (20) einen äußeren hohlen Stator (21) und einen zentrisch darin aufgenommenen und wenigstens teilweise hohl ausgebildeten rotierbaren Rotor (22) umfasst, wobei der Rotor (22) drehbar an dem Gehäuse (11) gelagert ist und eine Kopplungseinrichtung (23) zur drehfesten Kopplung mit der Antriebswelle (16) aufweist, um mittels der Antriebswelle (16) den wenigstens einen Rührflügel (13-15) anzutreiben, und dass der Stator (21) mit einer Mehrzahl von elektrischen Wicklungen (24) und der Rotor (22) mit einer Mehrzahl an Permanentmagneten (25) ausgerüstet ist,
und **dass** der Antriebsmotor (20) als Direktantrieb ausgebildet ist und die Antriebswelle getriebelos mit dem Antriebsmotor (20) verbunden ist, wobei ein Innendurchmesser (32) des hohlen Teils (33) des Rotors (22) wenigstens doppelt so groß ist wie ein Außendurchmesser (28) der Antriebswelle (16).

12. Fermenter (1) nach Anspruch 11, wobei der Rotor (22) innenliegend vorgesehen ist und der Stator (21) den Rotor (22) umgibt,
und/oder wobei die Antriebseinrichtung (12) über einen Frequenzumrichter (51) angesteuert wird,
und/oder wobei der Antriebsmotor (20) geeignet ist, bei einer Drehzahl von 1 Umdrehung/Sekunde ein Drehmoment von wenigstens 250 Nm und insbesondere von wenigstens 300 Nm aufzubringen,
und/oder wobei der Antriebsmotor (20) für Drehzahlen von bis zu 150 und insbesondere 200 Umdrehungen pro Minute und insbesondere für Drehzahlen zwischen 30 und 180 Umdrehungen pro Minute ausgebildet ist.

13. Fermenter (1) nach Anspruch 11 oder 12, wobei die Rühreinrichtung (10) automatisch höhenverstellbar und/oder seitenverstellbar ist.

## Claims

1. Agitating device (10) for a digester (1) of a biogas plant (100), having a housing (11) and a driving device (12) for rotatably driving at least one agitator blade (13-15), wherein the driving device (12) comprises a drive shaft (16) and an electric drive motor (20), wherein the drive motor (20) is accommodated sealed in the housing (11),
**characterized in**
**that** the drive motor (20) comprises an outer, hollow stator (21) and a rotary rotor (22) which is configured at least partially hollow, centrally accommodated therein, wherein the rotor (22) is rotatably supported on the housing (11) and comprises a coupling device (23) for non-rotatable coupling with the drive shaft (16) to drive the at least one agitator blade (13-15) by means of the drive shaft (16),
and **that** the stator (21) is equipped with a plurality of electric windings (24) and the rotor (22), with a plurality of permanent magnets (25), and that the drive motor (20) is configured as a direct drive, and the drive shaft (16) is coupled gearless with the drive motor (20), wherein the internal diameter (32) of the hollow part (33) of the rotor (22) is at least twice the size of the external diameter (28) of the drive shaft (16).

2. The agitating device (10) according to claim 1, wherein the coupling device (23) of the rotor (22) comprises a tooth flange (26), which comprises an internal toothing (27) to non-rotatably receive the drive shaft (16) equipped with an external toothing (17) .

3. The agitating device (10) according to any of the preceding claims, wherein the driving device (12) comprises an attachment device (30) having a bearing device (31) to rotatably support the drive shaft (16), wherein the attachment device (30) is detachably connected with the housing (11).

4. The agitating device (10) according to any of the preceding claims, wherein a blade hub (19) is non-rotatably disposed on the drive shaft (16), and the agitator blade (13-15) is attached to the blade hub (19),
and wherein the drive shaft (16) comprises in particular at least one radial engaging dog (18) for non-rotatable connection with the blade hub (19),
and/or wherein the blade hub (19) is axially fixed to the front end of the drive shaft (16) by means of a fixing unit (19a), and/or wherein the external diameter (29) of the blade hub (19) is at least twice, and in particular at least three times, the size of the external diameter (28) of the drive shaft (16).

5. The agitating device (10) according to any of the preceding claims, wherein the external diameter (34) of the rotor (22) is at least three times, and in particular at least four times, the size of the external diameter (28) of the drive shaft (16).

6. The agitating device (10) according to any of the preceding claims from claim 4, wherein the external diameter (34) of the rotor (22) is larger than the external diameter (29) of the blade hub (19).

7. The agitating device (10) according to any of the preceding claims, wherein the outer face of the stator (21) forms an outside surface of the housing (11).

8. The agitating device (10) according to any of the preceding claims, wherein the drive shaft (16) protrudes from a front side of the housing (11), and wherein a console accommodation (37) for attachment to a console (36) is disposed on the rear face of the housing (11).

9. The agitating device (10) according to any of the preceding claims, wherein the drive shaft (16) extends out of the housing (11) outwardly from the tooth flange (26) through a shaft opening (38) in an end cover (35), wherein at least one shaft seal (39) is disposed on the shaft opening (38) between the end cover (35) and the drive shaft (16).

10. The agitating device (10) according to any of the preceding claims, wherein a plurality of two, three or more agitator blades (11-13) is provided.

11. Digester (1) of a biogas plant (100) having a digester interior (3) that can be filled at least partially with a substrate (7), wherein at least one agitating device (10) controlled by a control device (50) is disposed in the digester interior (3), the agitating device (10) comprising a housing (11), at least one agitator blade (13-15) and a driving device (12) to rotatably drive the at least one agitator blade (13-15), the driving device (12) comprising a drive shaft (16) and an electric drive motor (20), which drive motor (20) is accommodated sealed in the housing (11),
**characterized in**
**that** the drive motor (20) comprises an outer, hollow stator (21) and a rotary rotor (22) which is configured at least partially hollow and is centrally accommodated therein, wherein the rotor (22) is rotatably supported on the housing (11) and comprises a coupling device (23) for non-rotatable coupling with the drive shaft (16) to drive the at least one agitator blade (13-15) by means of the drive shaft (16), and that the stator (21) is equipped with a plurality of electric windings (24) and the rotor (22), with a plurality of permanent magnets (25),
and **that** the drive motor (20) is configured as a direct drive, and the drive shaft (16) is coupled gearless with the drive motor (20), wherein the internal diameter (32) of the hollow part (33) of the rotor (22) is at least twice the size of the external diameter (28) of the drive shaft (16).

12. The digester (1) according to claim 11, wherein the rotor (22) is provided inside and the stator (21) surrounds the rotor (22), and/or wherein the driving device (12) is controlled by a frequency converter (51),
and/or wherein the drive motor (20) is suitable, given a speed of 1 revolution/second, to apply a torque of at least 250 Nm and in particular of at least 300 Nm,
and/or wherein the drive motor (20) is configured for speeds of rotation of up to 150 and in particular 200 revolutions per minute, and in particular for speeds of rotation between 30 and 180 revolutions per minute.

13. The digester (1) according to claim 11 or 12, wherein the agitating device (10) is automatically height-adjustable and/or side-adjustable.

## Revendications

1. Dispositif mélangeur (10) pour un fermenteur (1) d'une installation de méthanisation (100) doté d'un carter (11) et d'un dispositif entraîneur (12) destiné à entraîner en rotation au moins une aile agitatrice (13-15), ledit dispositif entraîneur (12) comprenant un arbre de transmission (16) et un moteur d'entraînement (20) électrique, ledit moteur d'entraînement (20) étant logé de manière hermétique dans ledit carter (11),
**caractérisé en ce**
**que** le moteur d'entraînement (20) comprend un stator (21) extérieur creux et un rotor (22) rotatif, réalisé au moins partiellement creux et logé axialement dans ledit stator,
ledit rotor (22) étant monté en rotation sur le carter (11) et présentant un dispositif de couplage (23) destiné à le coupler de manière rigide en rotation à l'arbre de transmission (16), afin d'entraîner à l'aide dudit arbre de transmission (16) ladite au moins une aile agitatrice (13-15), et que le stator (21) est équipé d'une pluralité de bobines électriques (24) et le rotor (22) d'une pluralité d'aimants permanents (25), et que le moteur d'entraînement (20) est réalisé en tant qu'entraînement direct et l'arbre de transmission est relié au moteur d'entraînement (20) sans engrenage, un diamètre intérieur (32) de la pièce creuse (33) du rotor (22) étant au moins deux fois plus grosse qu'un diamètre extérieur (28) de l'arbre de transmission (16).

2. Dispositif mélangeur (10) selon la revendication 1, le dispositif de couplage (23) du rotor (22) comprenant une bride dentée (26) qui présente une denture intérieure (27) destinée à recevoir, de manière rigide en rotation, l'arbre de transmission (16) équipé d'une denture extérieure (17).

3. Dispositif mélangeur (10) selon l'une quelconque des revendications précédentes, le dispositif entraîneur (12) comprenant un dispositif de montage (30) doté d'un dispositif support (31) destiné au montage en rotation de l'arbre de transmission (16), ledit dispositif de montage (30) étant relié de manière amovible au carter (11).

4. Dispositif mélangeur (10) selon l'une quelconque des revendications précédentes, un moyeu d'aile (19) étant agencé de manière fixe en rotation sur l'arbre de transmission (16) et l'aile agitatrice (13-15) étant fixée audit moyeu d'aile (19),
et l'arbre de transmission (16) comprenant notamment au moins une roue menante (18) radiale destinée à le relier de manière fixe en rotation au moyeu d'aile (19),
et/ou ledit moyeu d'aile (19) étant fixé axialement à l'aide d'une unité de fixation (19a) à l'extrémité avant de l'arbre de transmission (16),
et/ou un diamètre extérieur (29) du moyeu de l'aile (19) étant au moins deux fois plus grand et notamment au moins trois fois plus grand que le diamètre extérieur (28) de l'arbre de transmission (16).

5. Dispositif mélangeur (10) selon l'une quelconque des revendications précédentes, un diamètre extérieur (34) du rotor (22) étant au moins trois fois et notamment au moins quatre fois plus grand que le diamètre extérieur (28) de l'arbre de transmission (16).

6. Dispositif mélangeur (10) selon l'une quelconque des revendications précédentes à partir de la revendication 4, le diamètre extérieur (34) du rotor (22) étant supérieur à un diamètre extérieur (29) du moyeu de l'aile (19).

7. Dispositif mélangeur (10) selon l'une quelconque des revendications précédentes, la face externe du stator (21) constituant une face externe du carter (11).

8. Dispositif mélangeur (10) selon l'une quelconque des revendications précédentes, l'arbre de transmission (16) étant éloigné d'une face avant du carter (11) et un logement de console (37), qui est destiné à la fixation à une console (36), étant agencé sur la face arrière du carter (11).

9. Dispositif mélangeur (10) selon l'une quelconque des revendications précédentes, l'arbre de transmission (16) s'étirant de la bride dentée (26) vers l'extérieur en passant par une ouverture de l'arbre (38) dans un couvercle frontal (35) pour sortir du carter (11), au moins un joint d'étanchéité (39) étant agencé entre le couvercle frontal (35) et l'arbre de transmission (16) au niveau de l'ouverture de l'arbre (38).

10. Dispositif mélangeur (10) selon l'une quelconque des revendications précédentes, une pluralité de deux, trois ou plus ailes agitatrices (11-13) étant prévue.

11. Fermenteur (1) d'une installation de méthanisation (100) doté d'un espace intérieur de fermenteur (3) pouvant être rempli au moins partiellement de substrat (7), au moins un dispositif mélangeur (10) commandé par un dispositif de commande (50) étant agencé dans l'espace intérieur de fermenteur (3), le dispositif mélangeur (10) présentant un carter (11), au moins une aile agitatrice (13-15) et un dispositif entraîneur (12) destiné à entraîner en rotation ladite au moins une aile agitatrice (13-15), le dispositif entraîneur (12) comprenant un arbre de transmission (16) et un moteur d'entraînement (20) électrique, ledit moteur d'entraînement (20) étant logé de manière hermétique dans ledit carter (11),
**caractérisé en ce**
**que** le moteur d'entraînement (20) comprend un stator (21) extérieur creux et un rotor (22) rotatif, réalisé au moins partiellement creux et logé axialement dans ledit stator, ledit rotor (22) étant monté en rotation au carter (11) et présentant un dispositif de couplage (23) destiné à le coupler de manière rigide en rotation à l'arbre de transmission (16), afin d'entraîner à l'aide dudit arbre de transmission (16) ladite au moins une aile agitatrice (13-15), et que le stator (21) est équipé d'une pluralité de bobines électriques (24) et le rotor (22) d'une pluralité d'aimants permanents (25),
et **que** le moteur d'entraînement (20) est réalisé en tant qu'entraînement direct et l'arbre de transmission est relié au moteur d'entraînement (20) sans engrenage, un diamètre intérieur (32) de la pièce creuse (33) du rotor (22) étant au moins deux fois plus grand qu'un diamètre extérieur (28) de l'arbre de transmission (16).

12. Fermenteur (1) selon la revendication 1, le rotor (22) étant prévu à l'intérieur et le stator (21) entourant le rotor (22),
et/ou le dispositif entraîneur (12) étant piloté par le biais d'un variateur de vitesses (51), et/ou le moteur d'entraînement (20) étant apte à appliquer, pour une vitesse de rotation de 1 tour/seconde, un couple de rotation d'au moins 250 Nm et notamment d'au moins 300 Nm,
et/ou le moteur d'entraînement (20) étant conçu pour des vitesses de rotation allant jusqu'à 150 et notamment 200 tours par minute et notamment pour des vitesses de rotation entre 30 et 180 tours par minute.

13. Fermenteur (1) selon la revendication 11 ou 12, le dispositif mélangeur (10) étant automatiquement réglable en hauteur et/ou latéralement.
